# EUROPÄISCHE PATENTSCHRIFT

(11) **EP 2 209 554 B1**
(45) Veröffentlichungstag und Bekanntmachung des Hinweises auf die Patenterteilung: **11.10.2017**
(21) Anmeldenummer: 08849867.0
(22) Anmeldetag: 12.11.2008
(51) Int. Cl.: B01L 3/00

(54) **MODULARE SENSORKASSETTE**
MODULAR SENSOR CASSETTE
CASSETTE DE DÉTECTION MODULAIRE

(30) Priorität: 13.11.2007 US 987446 P
(43) Veröffentlichungstag der Anmeldung: 28.07.2010
(73) Patentinhaber: F. Hoffmann-La Roche AG, 4070 Basel (CH); Roche Diagnostics GmbH, 68305 Mannheim (DE)
(72) Erfinder: KONTSCHIEDER, Heinz, 8010 Graz (AT); LEINER, Marco Jean-Pierre, 8045 Graz (AT); HUBER, Wolfgang, 8502 Lannach (AT); KRYSL, Franz Josef, 8020 Graz (AT); RITTER, Christoph, 8045 Graz (AT); OFFENBACHER, Helmut, 8020 Graz (AT); SCHAFFAR, Bernhard, 8045 Graz (AT); SCHINNERL, Marie-Luise, 8102 Semriach (AT); HARER, Johann, 8042 Graz (AT)
(74) Vertreter: Babeluk, Michael
(86) Internationale Anmeldenummer: PCT/EP2008/065338
(87) Internationale Veröffentlichungsnummer: WO 2009/062940

(56) Entgegenhaltungen:
- WO-A-2008/001279
- DE-A1- 19 917 330
- DE-A1-102005 052 752
- US-A- 4 654 127

## Beschreibung

Die Erfindung betrifft eine Sensorkassette zum Einsetzen in einen Analysator, wobei die Sensorkassette einen durchgehenden Messkanal zur Aufnahme fluidischer Medien und sensorische Elemente zur Bestimmung von chemischen und/oder physikalischen Parametern der fluidischen Medien aufweist.

Messsysteme zur Bestimmung mehrerer Parameter in Körperflüssigkeiten stellen wichtige Bestandteile klinisch relevanter Analyseverfahren dar. Hierbei steht insbesondere eine schnelle und präzise Messung sogenannter Notfallparameter im Vordergrund.

Mit "Untersuchung am Ort der Versorgung" (Point-of-Care-Testing oder kurz POCT) bezeichnet man in der Medizin diagnostische Untersuchungen, die nicht in einem Zentrallabor, sondern im Krankenhaus unmittelbar auf der Krankenstation, in Intensivstationen, in der Anästhesie, aber auch in Ambulanzen, in der Dialyse, in der Praxis eines niedergelassenen Arztes, oder während eines Krankentransports durchgeführt werden. POCT hat den Vorteil, dass die Ergebnisse bereits nach kurzer Zeit vorliegen, da zum einen der Transport der Proben zu einem spezialisierten Labor entfällt und zum anderen keine Rücksicht auf die zeitlichen Abläufe des Labors genommen werden muss.

In der Regel handelt es sich um die Bestimmung sogenannter Notfallparameter, wie z.B. die Werte der Blutgase (O₂, CO₂), der pH-Wert, die Konzentrationen der Elektrolyte (Li⁺, Na⁺, K⁺, Mg⁺⁺, Ca⁺⁺, Cl⁻) die Konzentrationen der Metabolite (Glukose, Laktat, Harnstoff, Creatinin), die Werte der Hämoglobinderivate (O₂Hb, HHb, COHb, MetHb) und Bilirubin, der Hämatokritwert, die Bestimmung von Nierenfunktionswerten, Blutgerinnungswerten, Markern für kardiale Erkrankungen und andere Messwerte. Aber auch Urinuntersuchungen, die Erstellung eines Blutbildes oder der schnelle Nachweis von Krankheitserregern sind mit Hilfe von Point-of-Care-Methoden möglich.

Viele Point-of-Care-Untersuchungen sind als Teststreifen konzipiert. Sind jedoch mehrere Parameter gleichzeitig oder im Zusammenhang zu bestimmen, werden bevorzugt nahezu vollständig automatisierte Messgeräte bzw. Analysatoren eingesetzt, die in der Lage sind, ein ganzes Parameterpanel simultan zu bestimmen. Unter der Bestimmung eines Parameterpanels versteht man ganz allgemein die gemeinsame Bestimmung mehrerer Einzelparameter im Rahmen einer Messung. Bevorzugt werden hierbei Parameter zusammen bestimmt, welche entweder aufgrund gemeinsamer Messprinzipien leicht zusammen bestimmt werden können (z.B. Hämoglobinderivate anhand eines Messspektrums, verschiedene Elektrolyte oder Metabolite mittels analoger elektrochemischer oder optischer Nachweisverfahren) oder welche für eine diagnostische Bewertung der Analyseergebnisse in Zusammenhang stehen (z.B. Konzentrationen verschiedener kardialer Marker zur Differentialdiagnostik kardialer Erkrankungen oder Konzentrationen verschiedener Hämoglobinderivate zur Differentialdiagnostik bei Vorliegen von Gasstoffwechselstörungen).

Die Messungen erfolgen allgemein in austauschbaren Messkammern, die zumeist mit elektrochemischen (Elektroden) und/oder optischen (Optoden) sensorischen Elementen ausgestattet sind. Weiters werden hier auch photometrische/spektroskopische Methoden eingesetzt, wobei die optischen Eigenschaften der zu bestimmenden Probe oder Farbreaktionen zum Nachweis verwendet werden. In diesem Fall befinden sich im Probenkanal spezielle Bereiche, welche beispielsweise als optische Küvetten (optische Messfenster) ausgebildet sind, welche ebenfalls als sensorische Elemente im Sinne dieser Anmeldung angesehen werden können.

Die vorliegende Erfindung betrifft insbesondere solche Vorrichtungen, bei denen die Messkammer als Messkanal ausgebildet ist, in welche das zu untersuchende Medium, wie etwa Blut, eingebracht wird. Dabei kommt das zu untersuchende Medium in dieser Messkammer mit den sensorischen Elementen in Kontakt, um den eigentlichen Messvorgang zu ermöglichen. Dabei können mehrere unterschiedliche sensorische Elemente zu Gruppen sensorischer Elemente (Sensorarrays) zusammengefasst werden, welche in einem gemeinsamen Gehäuse oder auf einem gemeinsamen Träger angeordnet sind.

Aus der US 5,074,157 A (Marsoner) ist in diesem Zusammenhang ein Messkammerblock eines Analysators bekannt, der modulartig erweitert werden kann. Durch Ankopplung weiterer Module kann der Einsatzbereich des Gerätes vergrößert werden. Um die nötige Dichtheit der Verbindungsstellen der einzelnen Module zu gewährleisten, weisen die Kupplungsteile der Module Dichtringe auf. Von dem die einzelnen Messkammern verbindenden Messkanal zu den Kupplungsteilen des Moduls können Abzweigkanäle abführen, an welchen Kupplungsteilen weitere Messkammern ansteckbar sind. Dadurch wird eine Vergrößerung der Messstrecke erreicht, durch die es bei Bedarf möglich wird, das Parameterpanel zu erweitern. Um die Einhaltung vorgegebener Probentemperaturen sicherstellen zu können wird vorgeschlagen, die Module in einen thermostatisierbaren Aufnahmeblock einzusetzen. Die einzelnen Module sind lösbar miteinander verbunden und der durch die Module gebildete Messkammerblock ist nicht in Form einer austauschbaren Sensorkassette in die Aufnahme eines Analysators einsetzbar.

Die US 6,960,466 A (Pamidi et al.) beschreibt eine Sensorkassette, welche eine Anzahl einzelner Messelektroden zur Bestimmung unterschiedlicher Point Of Care Parameter wie Blutgase, Elektrolyte und Metabolite enthält, die auf einem gemeinsamen Träger aufgebracht sind.

Die EP 0 846 947 B1 (Huber et al.) beschreibt eine Sensorkassette mit planar ausgebildeten elektrochemischen und/oder optischen Sensoren, die auf einem gemeinsamen Sensorbauteil vorliegen.

Eine aus der DE 10 2005 052 752 A1 bekannte Vorrichtung weist eine Befülleinheit und eine Reaktionskartusche auf, wobei letztere einen Reaktionsraum beinhaltet. In einer Abbildungsfolge gemäß einer Ausführungsvariante wird dargelegt, dass sich die Reaktionskartusche und die Befülleinheit zunächst in einer Ausgangslage befinden, in welcher an den Bauteilen ausgebildete Rastelemente noch nicht im Eingriff stehen. Durch Zusammenpressen der beiden Teile kommt es zum Eindringen einer Befüllungskanüle und einer Abfallkanüle in den Reaktionsraum, welcher danach befüllt wird. Als letzter Schritt sind die Trennung der Kanäle und die Durchführung von Reaktionen bzw. deren Detektion dargestellt, wobei es wieder zu einer Auftrennung der Rastelemente kommt.

Die DE 199 17 330 A1 zeigt ein Mikroreaktormodul mit Reaktorelementen wie Fluidkanälen, Reaktionskammern, Heizvorrichtungen, Mischvorrichtungen und dergleichen, welches zur Ausbildung eines Mikrosystems aus einer Anzahl von Mikroreaktormodulen gleicher oder unterschiedlicher Art vorgesehen ist.

Schließlich ist aus der US 4,654,127 A eine Sensorkassette für einen klinischen Analysator bekannt, wobei die Sensorkassette auf einem Trägerteil ein Sensorarray aufweist, mit welchem klinische und/oder physikalische Parameter von Probenflüssigkeiten und Kalibriermitteln bestimmt werden können. Das Trägerteil bildet zusammen mit einem Abdeckteil einen Kapillarkanal aus, der eine Einlauföffnung und eine Auslauföffnung aufweist. Im Bereich der Einlauföffnung des Kapillarkanals ist am Abdeckteil eine Führungshülse ausgebildet, die einen drehbaren, zylindrischen Behälter aufnimmt, der in zwei durch eine Zwischenwand getrennte Kammern, eine Kalibriermittelkammer und eine Probenkammer unterteilt ist. Beide Kammern weisen am Boden Öffnungen auf, die zunächst mit jeweils einer Membran verschlossen sind. Beim Verdrehen des zylindrischen Proben/Kalibriermittelbehälters kann jeweils eine der Öffnungen mit der Einlauföffnung des Kapillarkanals verbunden werden, wobei die jeweilige Membran aufgerissen wird.

Nachteilig bei den bekannten Ausführungen ist, dass unterschiedliche sensorische Elemente, die beispielsweise unterschiedliche Betriebsbedingungen (z.B. unterschiedliche Betriebstemperatur) erfordern, auf einem einzigen Trägerteil oder Sensorbauteil vorliegen. Nachteilig ist auch, dass die gesamte Sensorkassette mit allen sensorischen Elementen als Ausschuss anfällt, wenn ein einziges sensorisches Element fehlerhaft ist. Nachteilig ist ferner die geringe Flexibilität bekannter Kassetten, wenn es um eine Verringerung oder Erweiterung des Parameterpanels geht. Für flexiblere Parameterpanels sind daher neue Lösungen anzustreben.

Die Lösung der gestellten Aufgabe führt zu einer Sensorkassette gemäß Patentanspruch 1, insbesondere zu einer Sensorkassette, die aus zumindest zwei fest miteinander verbundenen, jedoch separat hergestellten Sensormodulen besteht, welche jeweils ein Gehäuse und einen Messkanalabschnitt aufweisen, wobei die Messkanalabschnitte benachbarter Module durch eine fluidische Kopplung zum durchgehenden Messkanal verbunden sind und wobei jedes Sensormodul ein Sensorarray mit zumindest zwei, vorzugsweise planaren, sensorischen Elementen aufweist.

Weiterhin kann die Sensorkassette ein Dummymodul aufweisen, welches keine sensorischen Elemente, jedoch fluidische Anschlüsse an analogen Positionen wie das Sensormodul aufweist.

Weiterhin ist der erfindungsgemäßen Sensorkassette ein Speicherelement zugeordnet, auf welchem für die Sensorkassette spezifische Informationen, insbesondere zu deren Aufbau aus den jeweiligen Modulen, gespeichert sind. Über das gemeinsame Speicherelement sind die einzelnen Module der Sensorkassette zu einer integralen Einheit zusammengefasst.

Diese für die Sensorkassette spezifischen Informationen werden beim Einsetzen der erfindungsgemäßen Sensorkassette in den Analysator an diesen übertragen, beispielsweise durch spezielle im Analysator vorhandene Lesevorrichtungen. Dieses Einlesen der für die Sensorkassette spezifischen Informationen kann automatisiert (beispielsweise beim Einsetzen der Sensorkassette durch ein im Analysator integriertes Lesegerät) oder auch manuell (beispielsweise über Eingeben der Informationen über eine Eingabevorrichtung) erfolgen und übermittelt die für die Sensorkassette spezifischen Informationen so an den Analysator.

Als Speicherelement kann prinzipiell jede Vorrichtung eingesetzt werden, welche Informationen speichern und an einen Analysator bereitstellen kann. Bevorzugt werden als Speicherelemente Vorrichtungen eingesetzt, welche die für die Sensorkassette spezifischen Informationen automatisiert an eine entsprechende Lesevorrichtung des Analysators bereitstellen können. Solche bevorzugten Vorrichtungen können insbesondere elektronische Speicherelemente wie Memory Chips, bevorzugt wiederbeschreibbare Memory Chips, oder Speicherkarten (z.B. Flashspeicher) oder auch RFID-Transponder oder Magnetstreifen sein, welche beim Einsetzen der erfindungsgemäßen Sensorkassette in den Analysator die für die Sensorkassette spezifischen Informationen an eine entsprechende Lesevorrichtung im Analysator weiterleiten.

Weitere mögliche Speicherelemente sind optische Codes wie ein- oder zweidimensionale Barcodes, welche mittels eines Barcodescanners ebenfalls automatisiert eingelesen werden können.

Weiterhin ist es auch möglich, neben diesen automatisierten Übertragungsmöglichkeiten auch manuelle Eingaben der für die Sensorkassette spezifischen Informationen vorzusehen, beispielsweise können manuelle Eingaben solcher Informationen über eine Eingabeeinheit (Tastatur) des Analysators, erfolgen.

Gemäß der vorliegenden Erfindung ist jeder erfindungsgemäßen Sensorkassette ein Speicherelement zugeordnet, welches für die jeweilige Sensorkassette spezifische Informationen enthält. Diese Zuordnung erfolgt bevorzugt dadurch, dass das Speicherelement mit der Sensorkassette fest verbunden ist, so dass eine eindeutige Zuordnung gewährleistet ist. Dies kann beispielsweise dadurch erfolgen, dass das Speicherelement auf der Sensorkassette befestigt ist oder in die Sensorkassette integriert ist, beispielsweise durch Aufkleben eines Speicherelements oder Miteinbauen eines Speicherelements in die Sensorkassette bei Assemblieren dieser. Prinzipiell ist es auch möglich, das Speicherelement getrennt von der Sensorkassette anzuordnen, wobei in diesen Fällen durch andere Maßnahmen, beispielsweise durch identische Codierungen (z.B. Zahlencodes) auf Sensorkassette und Speicherelement gewährleistet werden muss, dass die Zuordnung von Sensorkassette und Speicherelement eindeutig ist, um die korrekten spezifischen Informationen der jeweiligen Sensorkassette zuzuordnen.

Als für die jeweilige Sensorkassette spezifische Informationen können allgemein alle Informationen angesehen werden, welche zumindest die Art und Weise des Aufbaus der Sensorkassette aus den einzelnen Modulen beschreiben.

Solche Informationen, welche den modularen Aufbau der Sensorkassette beschreiben, sind beispielsweise Informationen über die Art der verwendeten Module (z.B. Sensormodul oder Dummymodul; evtl. auch weitere Informationen über Typ des Moduls, z.B. elektrochemisches, optisches oder photometrisches/ spektroskopisches Messmodul oder die Verwendung des Messmoduls, z.B. Blutgasmodul, Elektrolytmodul, Metabolitmodul, Oxymetriemodul) und Informationen über die Anordnung bzw. Lage der einzelnen Module innerhalb der Sensorkassette, beispielsweise in welcher Reihenfolge die einzelnen Module entlang des durchgehenden Messkanals angeordnet sind.

In einer bevorzugten Ausführungsform umfassen die für jeweilige Sensorkassette spezifischen Informationen weiterhin Informationen, welche die Art und Weise der Anordnung der einzelnen sensorischen Elemente (oder auch freier Bereiche innerhalb der Module) in den jeweiligen Modulen und/oder deren Nutzung und Ansteuerung beschreiben.

Solche Informationen, welche den Aufbau und die Nutzung der einzelnen Module beschreiben, sind beispielsweise Informationen über die Anordnung, Ansteuerung und/oder Nutzung der einzelnen sensorischen Elemente innerhalb des jeweiligen Moduls.

Im Falle elektrochemischer Sensormodule können dies beispielsweise Informationen sein, welche die Anordnung und/oder Belegung der einzelnen (elektrischen) Kontaktstellen innerhalb eines Abgreifbereichs/Fensters des Sensormoduls beschreiben und beispielsweise beschreiben, ob und mit welchem elektrochemischen sensorischen Element eine solche Kontaktstelle verbunden ist und wie diese zu nutzen ist.

Im Falle optischer Sensormodule können dies beispielsweise Informationen sein, welche die Anordnung und/oder Belegung der einzelnen signalabgreifenden Bereiche innerhalb eines Abgreifbereichs/Fensters des Sensormoduls beschreiben und beispielsweise beschreiben, ob und mit welchem optischen sensorischen Element ein solcher signalabgreifender Bereich verbunden ist und wie dieser zu nutzen ist.

Im Falle photometrischer/spektroskopischer Sensormodule können dies beispielsweise Informationen sein, welche die Anordnung einzelner optischer Messfenster oder Küvetten innerhalb des Messkanals und/oder deren Nutzung innerhalb des jeweiligen Moduls beschreiben.

Neben diesen Informationen über Art und Weise der Anordnung der einzelnen sensorischen Elemente in den jeweiligen Modulen und/oder deren Nutzung und Ansteuerung können auch modulspezifische Informationen enthalten sein, welche die Anordnung und Nutzung einzelner fluidischer Anschlüsse innerhalb des jeweiligen Moduls beschreiben.

So können beispielsweise Informationen zu den jeweiligen Modulen enthalten sein, welche Art bzw. Nutzung die einzelnen fluidischen Anschlüsse beschreiben (z.B. Fluidanschlüsse des Messkanals (Eingangsanschluss der Sensorkassette, Ausgangsanschluss der Sensorkassette, Verbindungsanschlüsse zwischen den einzelnen Modulen) oder fluidische Neben- oder Hilfsanschlüsse (z.B. zur Zuführung des Referenzelektrolyten bei elektrochemischen Messmodulen oder zur Zufuhr zur Analytbestimmung benötigter Reagenzien) oder auch Leer- oder Blindanschlüsse (z.B. bei Dummymodulen).

Weiterhin können auch modulspezifische Informationen enthalten sein, welche die Anordnung und Nutzung bestimmter thermischer Kontaktzonen innerhalb des jeweiligen Moduls beschreiben.

So können beispielsweise Informationen zu den jeweiligen Modulen enthalten sein, welche die Ansteuerung die einzelnen thermischen Kontaktzonen durch den Analysator beschreiben, beispielsweise Informationen, welche thermische Kontaktzone auf welche Temperatur zu thermostatisieren ist. Alternativ können sich auch Temperiereinrichtungen innerhalb der Module selbst befinden. In diesem Falle können Informationen zu den jeweiligen Modulen enthalten sein, welche die Anordnung der zu deren Ansteuerung notwendigen modulseitigen elektrischen Kontaktstellen und/oder deren dementsprechende Nutzung beschreiben.

Weiterhin können auch modulspezifische Informationen enthalten sein, welche die Anordnung und Nutzung bestimmter fluidischer Steuerelemente (beispielsweise Pumpen oder Ventile) innerhalb des jeweiligen Moduls beschreiben.

So können beispielsweise Informationen zu den jeweiligen Modulen enthalten sein, welche die Nutzung und Ansteuerung der einzelnen fluidischen Steuerelemente durch analysatorseitige oder modulseitige Aktuatoren beschreiben.

Diese auf das jeweilige Modul gerichteten Informationen sind vorzugsweise zumindest teilweise auf dem Speicherelement gespeichert und können so zusammen mit den für die Sensorkassette spezifischen Informationen an den Analysator übermittelt werden. Alternativ ist es auch möglich, dass alle oder ein Teil dieser modulbezogenen Informationen bereits in einem Speicher des Analysators hinterlegt sind, so dass in Kenntnis der zumindest auf dem Speicherelement enthaltenen und von diesem an den Analysator übertragbaren Informationen, welche den modularen Aufbau der Sensorkassette beschreiben, eine entsprechende Verknüpfung dieser Informationen erfolgen kann und so die zum korrekten Betrieb der Sensorkassette notwendigen Informationen im Analysator vorliegen.

In einer weiterhin bevorzugten Ausführungsform umfassen die für jeweilige Sensorkassette spezifischen Informationen weiterhin Informationen, welche die Art der einzelnen sensorischen Elemente und/oder deren Nutzung und Ansteuerung beschreiben.

Solche Informationen umfassen beispielsweise alle Informationen, welche zum Betrieb des sensorischen Elements benötigt werden und/oder der Ermittlung der mit dem sensorischen Element zu bestimmenden Parameter verwendet werden. Solche Informationen werden austauschbaren sensorischen Elementen häufig standardmäßig in Form gespeicherter Daten mitgegeben und umfassen beispielsweise Informationen zu Art des sensorischen Elements, Herstellinformationen (z.B. Chargennummer), Kennliniendaten und/oder Kalibrationsinformationen oder Haltbarkeitsinformationen (z.B. Lebensdauer, Ablaufdatum, Zahl der möglichen Messungen) des jeweiligen sensorischen Elements.

Diese auf das jeweilige sensorische Element gerichteten Informationen sind vorzugsweise zumindest teilweise auf dem Speicherelement gespeichert und können so zusammen mit den für die Sensorkassette spezifischen Informationen und optional auch mit weiteren auf das jeweilige Modul gerichteten Informationen an den Analysator übermittelt werden. Alternativ ist es auch möglich, dass alle oder ein Teil dieser auf das jeweilige sensorische Element bezogenen Informationen bereits in einem Speicher des Analysators hinterlegt sind, so dass in Kenntnis der zumindest auf dem Speicherelement enthaltenen und von diesem an den Analysator übertragbaren Informationen, welche den modularen Aufbau der Sensorkassette beschreiben (und optional auch mit weiteren das jeweilige Modul gerichteten Informationen), eine entsprechende Verknüpfung dieser Informationen erfolgen kann und so die zum korrekten Betrieb der Sensorkassette notwendigen Informationen im Analysator vorliegen.

Die erfindungsgemäße Kassette kann zumindest zwei Sensormodule und zumindest ein Dummymodul aufweisen, welches bis auf die fehlenden sensorischen Elemente im Wesentlichen mit dem jeweiligen Sensormodul baugleich ausgeführt ist. Das Dummymodul unterscheidet sich vom Sensormodul somit dadurch, dass es keine sensorischen Elemente aufweist, ansonsten aber im Wesentlichen gleich aufgebaut ist und beispielsweise fluidische und elektrische Anschlüsse an analogen Positionen wie ein Sensormodul ausweist. Die Außenabmessungen der Sensorkassette können dadurch konstant gehalten werden.

Besonders vorteilhaft weisen die aus verschiedenen Modulen aufgebauten Kassetten, die in einem Analysatortyp verwendet werden, kompatible Abmessungen und Anschlusszonen auf. Der modulare Aufbau gewährleistet eine hohe Flexibilität. Beispielsweise können neue Parameter und Parameterpanels auch nach der Markteinführung eines Analysators entwickelt werden, ohne dass die Hardware der bereits am Markt befindlichen Geräte umgerüstet werden muss. Weiterhin können verschiedene Module, welche verschiedene Parameter oder Parameterpanels bestimmen können, in verschiedenen Konfigurationen zusammengestellt werden, so dass dem Benutzer auf einfache Weise verschiedene, auf dessen Bedürfnisse abgestimmte Sensorkassetten angeboten werden können. Durch diesen modularen Aufbau können die Herstellkosten solcher unterschiedlicher Sensorkassetten deutlich reduziert werden. Weiterhin ist es auch möglich, zur Bestimmung des gleichen Parameters Sensormodule mit unterschiedlichen sensorischen Elementen zur Bestimmung dieses Parameters zu verwenden, welche beispielsweise auf unterschiedlichen sensorischen Prinzipien beruhen oder unterschiedliche Konzentrationsbereiche eines Analyten abdecken können.

Alternativ zur Probeneingabe durch eine im Analysator befindliche Vorrichtung kann die Probeneingabe auch durch ein spezielles, mit der modularen Kassette verbundenes Modul (Probeeingabe-Modul) erfolgen.

Ein Sensormodul einer erfindungsgemäßen Sensorkassette besteht beispielsweise aus:
a) einem Trägerteil, d.h., einem Sensorbauteil, auf dem ein sensorisches Element oder auch eine Reihe sensorischer Elemente (Sensorarray) aufgebracht sind,
b) einem Abdeckteil, in dem ein durchgängiger Messkanal bzw. Messkanalabschnitt ausgeformt ist, der für den Durchfluss fluidischer Medien bestimmt ist,
c) einem Dichtelement zur Abdichtung des Messkanals, wobei das Dichtelement zwischen dem Trägerteil und dem Abdeckteil vorliegt oder am Abdeckteil angeformt ist,
d) einer ersten Öffnung an einem Ende des Messkanals zum Anschluss eines weiteren Moduls,
e) einer zweiten Öffnung am anderen Ende des Messkanals zum Anschluss an ein Analysegerät oder ein weiteres Modul.

Die Verbindung zwischen dem Sensorbauteil und dem Abdeckteil erfolgt
- mit einem Dichtelement

An den Verbindungsstellen der Messkanalabschnitte der einzelnen Module können sich weitere Dichtelemente befinden. Der feste Zusammenhalt der Module kann durch mechanische Verschnappung, Verschweißung oder Verklebung erfolgen. Unter festem Zusammenhalt der Module wird insbesondere verstanden, dass die einzelnen Module bei der Herstellung der Sensorkassette in einer Art und Weise verbunden werden, dass diese eine nicht mit einfachen Mitteln, vorzugsweise nicht ohne Zerstörung der Sensorkassette, vom Benutzer der Sensorkassette getrennt werden können. Für den Benutzer stellt die modular aufgebaute Sensorkassette somit funktionell ein einziges in den Analysator einzusetzendes Bauteil (Consumable) dar.

Die modulare Sensorkassette verfügt über zumindest zwei fluidische Anschlussstellen zum Analysator für den Einlass bzw. Auslass von Probenmedien und Funktionsfluide (z.B. Kalibrier-, QC- und Waschfluide) und ggf. weitere fluidische Anschlussstellen zum Analysator für Hilfsfluide (z.B. Innenelektrolyt für Referenzelektroden oder Reagenzien).

Im Prinzip können die sensorischen Elemente für die einzelnen zu bestimmenden Parameterwerte über die verschiedenen Sensormodule verteilt werden. Bevorzugt gibt es jedoch für jede der Parametergruppen Blutgase, Elektrolyte, Metabolite usw. oder auch sonstige Parameterpanels je ein separates Sensormodul.

Von einzelnen Sensormodulen kann es mehrere Varianten geben. So enthält z.B. eine erste Variante eines Moduls zur Bestimmung der Elektrolytwerte sensorische Elemente zur Messung des vollständigen Elektrolytpanels (Li⁺, Na⁺, K⁺, Ca²⁺, Cl⁻. Mg²⁺). Eine zweite Variante enthält z.B. sensorische Elemente zur Messung des am häufigsten benötigten Elektrolytpanels (Na⁺, K⁺, Ca²⁺, Cl⁻).

Analog enthält eine erste Variante des Moduls zur Bestimmung von Metabolitwerten sensorische Elemente zur Messung eines breiten Metabolitpanels (Glukose, Laktat, Harnstoff, Kreatinin). Eine zweite Variante des Moduls enthält sensorische Elemente zur Messung der am häufigsten benötigten Metabolite (Glukose, Laktat).

Dies hat den Vorteil, dass werkseitig nur jene sensorischen Elemente gefertigt werden müssen, die auch tatsächlich verwendet werden. Insbesondere bei der Fertigung von Sensorelementen, die wie insbesondere chemischen Sensorelemente aus unterschiedlichen Materialien bestehen und in einer Vielzahl von Fertigungsschritten hergestellt werden, sind in der Regel die Ausschussraten hoch, wenn sämtliche sensorische Elemente in ein einziges Sensorbauteil integriert werden. In der Regel ist die Fertigung eines sensorischen Elementes mit einer gewissen Ausschussrate behaftet. Bei der Fertigung mehrerer sensorischer Elemente auf einem einzigen Sensorbauteil erhöht sich die Ausschussrate entsprechend. Umfasst eine Sensorkassette eine Vielzahl unterschiedlicher Parameter, ist die Fertigung und das Assemblieren vieler Sensorbauteile mit je einem sensorischen Element ebenfalls aufwendig. So hat sich das Zusammenfassen sensorischer Elemente zu Gruppen von sensorischen Elementen auf möglichst wenigen Sensorbauteilen als besonders vorteilhaft herausgestellt. Dabei ist es besonders vorteilhaft, wenn ähnlich aufgebaute und mit identischen Fertigungsschritten und Fertigungsverfahren herstellbare sensorische Elemente, wie z.B. die Gruppe der Blutgaselektroden, die Gruppe der ionenselektiven Elektroden, die Gruppe der amperometrischen Biosensoren jeweils auf einem Substrat gefertigt werden. Analoges gilt für optische Sensoren. Solche Anordnungen für optische Sensoren sind beispielsweise in US 5,351,563 oder US 6,652,810 dargestellt.

Schließlich können auch Sensormodule für photometrische/spektroskopische Analyseverfahren vorgesehen sein. Solche Sensormodule enthalten innerhalb des Probenkanals (oder zumindest mit diesem fluidisch verbunden, beispielsweise in einem Seitenkanal angeordnet) spezielle Bereiche, welche als optische Küvetten (optische Messfenster) ausgebildet sind, welche ebenfalls als sensorische Elemente im Sinne dieser Anmeldung angesehen werden können. Beispiele für derartige Nachweisverfahren und sensorischen Prinzipien sind die Bestimmung der Hämoglobinderivate und Bilirubin oder photometrische HbA1c-Methoden. Beispielhafte Anordnungen hierfür sind unter anderem in EP 1 445 020 A1, US 6,582,964 oder US 6,388,752 beschrieben.

Weiters ist es möglich, über weitere Anschlussstellen an einem Modul Reagenzien zuzusetzen, wonach eine Nachweisreaktion, beispielsweise eine Farbreaktion, ausgelöst wird und die entsprechenden Parameter beispielsweise photometrisch bestimmt werden. Beispiele sind photometrische HbA1c-Bestimmungsmethoden.

Weitere Sensormodule können auf immunologischen Methoden basierende sensorische Elemente enthalten, beispielsweise zur Bestimmung bestimmter Cardiac Marker wie NTproBNP oder Troponin. Bei solchen immunologischen Nachweismethoden ist ein Zusatz weiterer Reagenzien (z.B. Antikörper, Labels, Waschlösungen) zur Analytbestimmung notwendig, welche entweder durch zusätzliche fluidische Anschlüsse (im Sinne von fluidischen Hilfsanschlüssen) in das jeweilige Sensormodul eingebracht werden können oder bereits in diesem Sensormodul vorliegen. Die sensorischen Nachweisprinzipien können hier photometrische oder spektroskopische Nachweisverfahren sein (z.B. Nachweis mittels gold- oder farbstoffgelabelter Antikörper), aber auch andere Nachweisverfahren sind grundsätzlich denkbar.

Weiters kann auch ein Modul zur Bestimmung von Gerinnungsparametern vorgesehen sein. Auch bei der Bestimmung von Gerinnungsparametern, welche zumeist auf enzymatischen Reaktionen beruhen, ist ein Zusatz weiterer Reagenzien (z.B. gelabelte spezifische Substrate) zur Analytbestimmung notwendig, welche entweder durch zusätzliche fluidische Anschlüsse (im Sinne von fluidischen Hilfsanschlüssen) in das jeweilige Sensormodul eingebracht werden können oder bereits in diesem Sensormodul vorliegen. Die sensorischen Nachweisprinzipien können hier photometrische oder spektroskopische Nachweisverfahren sein (z.B. Nachweis farbiger Reaktionsprodukte einer enzymatischen Reaktion), aber auch andere Nachweisverfahren (z.B. Nachweis elektrochemisch aktiver Reaktionsprodukte eine enzymatischen Reaktion mittels elektrochemischer Nachweisverfahren) sind grundsätzlich denkbar.

Die Erfindung wird im Folgenden anhand von Zeichnungen näher erläutert. Es zeigen:
- Fig. 1: eine erfindungsgemäße, modulare Sensorkassette mit drei Sensormodulen in einer schematischen Draufsicht;
- Fig. 2: eine Variante der Sensorkassette gemäß Fig. 1 mit zwei zu einem Doppelmodul zusammengefassten Sensormodulen;
- Fig. 3: eine Variante der Sensorkassette gemäß Fig. 1, bei welcher ein Sensormodul durch ein Dummymodul ersetzt ist;
- Fig. 4: eine Variante der Sensorkassette gemäß Fig. 2, bei welcher in einem Doppelmodul ein von Sensorelementen freier Bereich D ausgeführt ist,
- Fig. 5: ein konkretes Ausführungsbeispiel einer modulare Sensorkassette in dreidimensionaler Darstellung;
- Fig. 6: die modulare Sensorkassette gemäß Fig. 5 in einer Explosionsdarstellung; sowie
- Fig. 7: ein weiteres, konkretes Ausführungsbeispiel einer modulare Sensorkassette in dreidimensionaler Darstellung

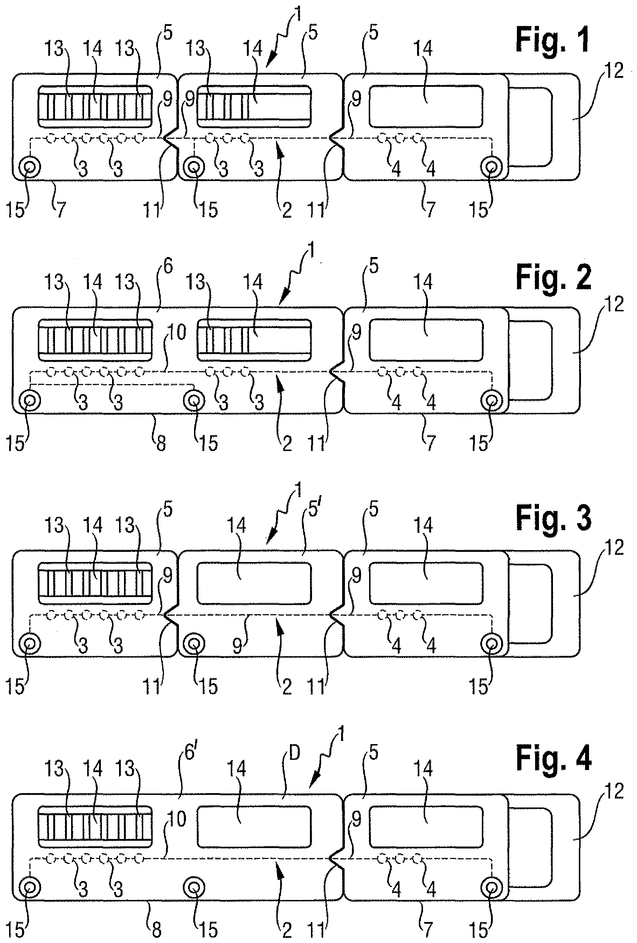

Die in Fig. 1 schematisch dargestellte Sensorkassette 1 ist in die Aufnahme eines nicht weiter dargestellten Analysators einsetzbar und weist einen durchgehenden Messkanal 2 (strichliert dargestellt) auf, der zur Aufnahme fluidischer Medien, wie beispielsweise Probenflüssigkeiten, Kalibrier- Qualitätskontroll- und Waschmedien dient. In der Sensorkassette 1 sind sensorische Elemente 3, 4 zur Bestimmung von chemischen und/oder physikalischen Parametern der fluidischen Medien angeordnet. Die Sensorkassette 1 besteht gemäß Fig. 1 aus drei fest miteinander verbundenen, äußerlich im Wesentlichen gleichartig aufgebauten Modulen 5, die in einem Gehäuse 7 jeweils Messkanalabschnitte 9 aufweisen, wobei die Messkanalabschnitte benachbarter Module durch eine fluidische Kopplung 11 zum durchgehenden Messkanal 2 verbunden sind. Jedes der Module 5 ist im dargestellten Beispiel als Sensormodul für unterschiedliche Arten von sensorischen Elementen ausgeführt, wobei die ersten beiden Module elektrochemische Sensoren 3 und das letzte Modul 5 mit dem Griffelement 12 beispielsweise optische Sensoren 4 aufweist.

Bei den Modulen 5 mit den elektrochemischen Sensoren 3 sind elektrische Kontaktstellen 13 angedeutet, die durch ein Fenster 14 im Modul 5 durch entsprechende Kontaktstifte des Analysators kontaktiert werden können. Der Sachverhalt entspricht in diesem Zusammenhang im Wesentlichen jenem der eingangs zitierten EP 0 846 947 B1. Die Fluidanschlüsse der Sensorkassette 1 zum Analysator sind mit 15 bezeichnet. Bei dem letzten Modul 5 mit dem Griffelement 12, welches optische Sensoren 4 enthält, sind innerhalb des Fensters 14 (nicht explizit dargestellt) signalabgreifenden Bereiche ausgebildet, welche jeweils mit einem optischen Sensor 4 verbunden sind und dessen jeweilige Antwort zur Parameterbestimmung an den Analysator weiterleiten.

Aus der Gegenüberstellung der Ausführungsvarianten der Sensorkassette 1 gemäß Fig. 1 bis Fig. 4 ist erkennbar, dass die äußeren Abmessungen der einzelnen Varianten trotz unterschiedlicher Sensormodule kompatibel sind, was insbesondere auch für die Form und die Lage der Fluidanschlüsse 15 gilt. Im in Fig. 1 dargestellten Fall sind die fluidischen Anschlüsse 15 der beiden äußeren Module als sensorkassettenseitiger Ein- bzw. Ausgang des durchgehenden Messkanals 2 ausgebildet, welche den fluidischen Anschluss an den Analysator zum Zwecke des Einbringens von Probenflüssigkeiten und/oder Funktionsfluiden ermöglichen. Der fluidische Anschluss 15 des mittleren Sensormoduls kann hier als fluidischer Neben- oder Hilfsanschluss (z.B. zur Zuführung des Referenzelektrolyten bei elektrochemischen Messmodulen oder zur Zufuhr zur Analytbestimmung benötigter Reagenzien) dienen.

So können beispielsweise gemäß der Ausführungsvariante Fig. 2 zwei Einzelmodule zu einem längeren Modul 6 mit einem Gehäuse 8 zusammengefasst sein, wobei das Modul 6 die doppelte Länge der Module 5 aufweist. In der hier gezeigten Anordnung ist einer der beiden fluidischen Anschlüsse 15 des Moduls 6 als sensorkassettenseitiger Ein- bzw. Ausgang des durchgehenden Messkanals 2 ausgebildet, während der andere fluidische Anschluss hier als fluidischer Neben- oder Hilfsanschluss (z.B. zur Zuführung des Referenzelektrolyten bei elektrochemischen Messmodulen oder zur Zufuhr zur Analytbestimmung benötigter Reagenzien) ausgebildet.

Ein Modul kann prinzipiell auch sensorische Elemente enthalten, welche auf unterschiedlichen Nachweisprinzipien beruhen. Beispielsweise könnte in Fig. 2 das links dargestellte Modul in dessen linkem Bereich elektrochemische sensorische Elemente enthalten und im rechten Bereich andere sensorische Elemente, beispielsweise optische sensorische Elemente enthalten.

Weiters kann gemäß der Ausführungsvariante in Fig. 3 die Sensorkassette 1 zwei Sensormodule 5 und (in der Mitte) ein Dummymodul 5' aufweisen, welches bis auf die fehlenden sensorischen Elemente im Wesentlichen den Sensormodulen 5 entspricht. In diesem Fall ist der fluidische Anschluss 15 des Dummymoduls 5' als Leer- oder Blindanschluss ausgebildet.

Wie in Fig. 4 dargestellt, ist es auch möglich, in der Sensorkassette 1 ein Modul 6' vorzusehen, welches ein Sensorarray mit sensorischen Einheiten 3 und einem freien Bereich D aufweist, der frei von sensorischen Elementen ist. Durch diese Bauweise werden selbst bei geänderter Bestückung mit sensorischen Elementen gleiche Außenabmessungen verwirklicht. Das Dummymodul 5' (Fig. 3) bzw. der freien Bereich D des Moduls 6' (Fig. 4) kann einen Fluidanschluss 15 aufweisen, der als Leeranschluss ausgeführt ist.

Erfindungsgemäß weisen die Sensormodule 5, 6, 6' und die Dummymodule 5' einer Sensorkassette 1 gemeinsam die der Aufnahme des Analysators entsprechenden Abmessungen und Fluidanschlüsse 15 auf. Ähnliches gilt auch für die elektrischen Kontaktstellen 13 oder sonstige signalabgreifende Bereiche innerhalb der Fenster 14.

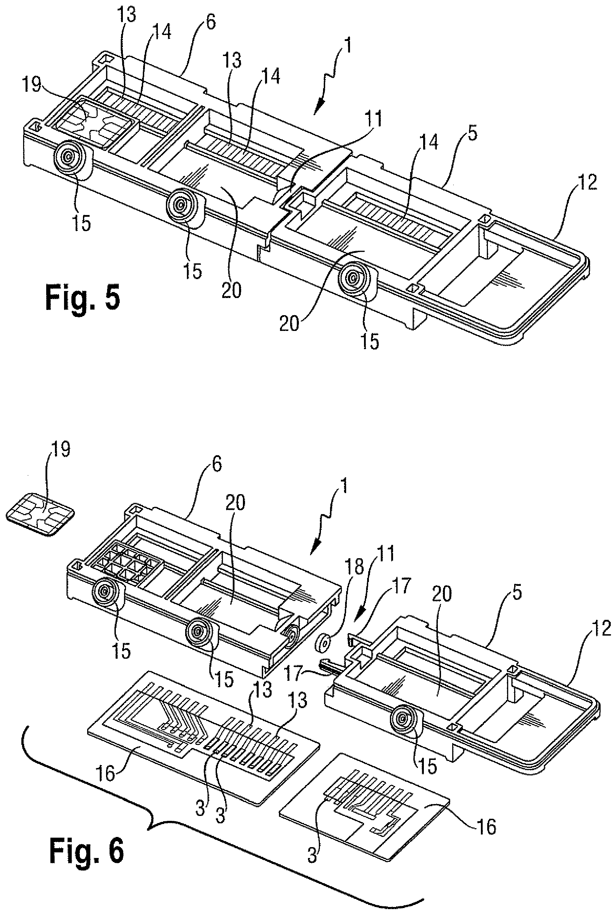

In den Fig. 5 und Fig. 6 ist ein konkretes Ausführungsbeispiel einer modularen Sensorkassette 1 dargestellt, die aus einem Einfachmodul 5 (mit hier fest verbundenem Griffteil 12) und einem Doppelmodul 6 mit zwei Fluidanschlüssen 15 besteht. Insbesondere aus Fig. 6 ist erkennbar, dass auf einem Trägerteil 16 eine Reihe elektrochemischer Sensorelemente 3 aufgebracht sind, welche über Leiterbahnen mit den Kontaktstellen 13 verbunden sind. Die elektrischen Kontakte werden vom Analysator durch das Fenster 14 der Module 5, 6 abgegriffen.

Die einzelnen Module 5, 6 der Sensorkassette 1 sind durch eine feste mechanische Verschnappung, beispielsweise mittels Rastelementen 17, unter Zwischenlage einer Dichtung 18 miteinander verbunden. Die feste Verbindung kann auch durch eine Verschweißung oder durch eine Verklebung hergestellt sein.

Auf einem der Module 5, 6 der Sensorkassette 1 ist ein Speicherelement 19 als Speicherchip angeordnet, auf welchem für die Sensorkassette 1 spezifische Informationen, insbesondere zu deren Aufbau aus den jeweiligen Modulen 5, 6, gespeichert sind, die nach dem Einsetzen der Sensorkassette 1 in den Analysator automatisch ausgelesen werden.

Der Trägerteil 16 (beispielsweise dessen Rückseite) kann als thermische Kontaktzone dienen, über welche die einzelnen Module 5, 6 im Analysator auf eine unterschiedliche Betriebstemperatur thermostatisiert werden können. Wie aus Fig. 6 erkennbar, können die thermischen Kontaktzonen benachbarter Module 5, 6 der Sensorkassette 1 thermisch entkoppelt sein (z.B. durch einen entsprechenden Abstand der Trägerteile 16 oder durch temperaturisolierende Materialien).

Die Sensormodule 5, 6, 6' können optische Durchtrittszonen für Anregungs- und Messstrahlung aufweisen, falls optische Sensoren 4 im Modul vorliegen. Zusätzlich können optische Fenster (Küvetten) für Transmissions- oder Reflektionsmessungen vorgesehen sein. Damit können beispielweise mittels spektroskopischer Methoden Hämoglobinwerte bestimmt werden.

Auch die Dummymodule 5' können thermische Kontaktzonen zum Vorwärmen bzw. Abkühlen von Probe und Funktionsfluiden enthalten, um diese beispielsweise auf eine zu nachfolgende Sensormodule notwendige Betriebstemperatur zumindest teilweise vorzuwärmen. Alternativ können sich auch Temperiereinrichtungen innerhalb der Module selbst befinden, welche beispielsweise durch entsprechende elektrische Kontaktierung von Analysator entsprechend angesteuert werden.

Die Sensormodule 5, 6, 6' können so auf unterschiedlicher Betriebstemperatur thermostatisiert werden, z.B. können die Sensoren eines ersten Moduls bei Körpertemperatur (z.B. Blutgassensoren bei 37°C) und die Sensoren eines zweiten Moduls bei einer niedrigeren Temperatur betrieben werden (z.B. Metabolit- oder Elektrolyt-Sensoren bei 30 °C).

Dadurch ist bei den empfindlichen biochemischen Sensoren eine höhere Betriebsstabilität erreichbar.

Besonders vorteilhaft für den Betrieb bei unterschiedlichen Temperaturen ist es, wenn die Sensormodule 5, 6, 6' an den Kontaktstellen zumindest teilweise thermisch entkoppelt sind, beispielweise durch Materialien mit einer niedrigen Wärmeleitung.

Einzelne Module der Sensorkassette können auch Bereiche mit in den Fluidwegen integrierten Fluidikelementen oder- funktionalitäten aufweisen, welche beispielsweise Ventilfunktionen oder Pumpenfunktionen umfassen. Auf das durch die Fluidwege fließende Fluid wird hierdurch mittels entsprechender Aktuatoren eingewirkt. So können beispielsweise Ventile eingesetzt werden, über die die Fluidwege durchgeschaltet oder verschlossen werden. Zum anderen können in einzelnen Modulen Pumpen zur Förderung des Fluids integriert werden. Hierbei ist es nicht unbedingt notwendig, dass alle Bestandteile der Fluidikelemente oderfunktionalitäten in dem Modul enthalten sind. Bestimmte Bestandteile dieser Fluidikelemente oder- funktionalitäten können auch analysatorseitig angeordnet sein und greifen dann in entsprechende modulseitige Teilelemente ein, um im Zusammenspiel eine fluidische Wirkung hervorzurufen. Beispiel hierfür sind Peristaltikpumpen mit analysatorseitigen Rotor und modulseitigem Schlauch oder auch Sperrventile mit analysatorseitig angeordneten Stößeln und entsprechenden modulseitig angeordneten quetschbaren Schlauchabschnitten.

Bevorzugt wird die modulare Sensorkassette im Werk assembliert und für den Anwender gebrauchsfertig in geeigneten Gebinden verpackt.

Beschreibung beispielhafter Sensorkassetten:

### Beispiel 1:

Die modulare Sensorkassette 1 zur Bestimmung von Blutwerten (siehe Fig. 5 und Fig. 6) wird mit zwei Sensormodulen 5, 6 realisiert, die werkseitig durch eine Schnappverbindung untrennbar verbunden werden. Das erste Modul 5 der Sensorkassette enthält sensorische Elemente zur Bestimmung der Blutgas-Parameter (pO₂, pCO₂) dem pH-Wert und dem Hämatokrit, das zweite Modul 6 enthält sensorische Elemente zur Bestimmung von Metabolitwerten (Glukose, Laktat,) und Elektrolytkonzentrationen (Na⁺, K⁺, Ca²⁺, Cl⁻.). Das Modul 6 enthält ferner eine Referenzelektrode (hier durch die beiden linken der auf dem Träger 16 des Moduls 6 angebrachten Leiterbahnen gebildet), die über den rechten fluidischen Anschluss 15 des Moduls 6 vom Analysator mit einem Funktionsfluid (= Innenelektrolytflüssigkeit) versorgt wird.

Die Sensormodule 5, 6 haben in diesem Beispiel unterschiedliche geometrische Abmessungen.

Nicht explizit dargestellt sind in dieser Figur Dichtelemente zwischen den Trägerteilen 16 und den entsprechenden Gehäuseoberteilen der jeweiligen Module. Diese Dichtelemente definieren in Zusammenspiel mit den jeweiligen Trägern und Gehäuseoberteilen die fluidischen Kanäle, insbesondere auch den Messkanal, innerhalb der jeweiligen Module.

Das zweite Modul 6 ist annähernd doppelt so lang wie das erste Modul 5 (abzüglich Griffteil 12). Beide Module werden mit unterschiedlichen Temperaturen (37°C bzw. 30°C) betrieben.

### Beispiel 2:

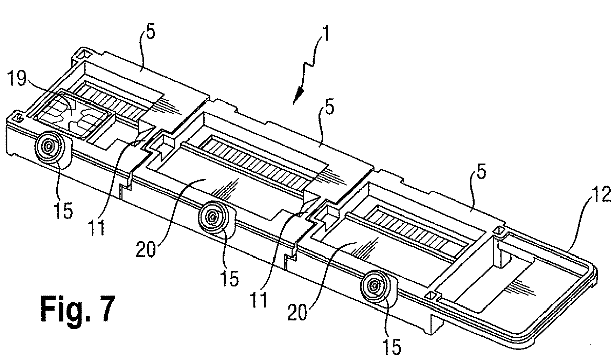

Alternativ kann das zweite Modul 6 aus Beispiel 1 durch zwei kurze Module 5 ersetzt werden (siehe Fig. 7), wobei eines die Elektrolytsensoren und das andere die Metabolitsensoren beinhaltet. Man erhält dadurch eine erfindungsgemäße modulare Sensorkassette 1 mit einem Blutgas-Modul (mit Griffteil 12), einem Elektrolyt-Modul (in der Mitte) und einem Metabolit-Modul. Die Lage und Verwendung der fluidischen Anschlüsse 15 ist hier lediglich schematisch dargestellt. Prinzipiell unterliegt die Art und Anordnung der Module innerhalb der Sensorkassette keinen Beschränkungen. So können neben Sensormodulen und Dummymodulen prinzipiell noch weitere Module eingefügt werden, welche spezielle Funktionen übernehmen können. So können beispielsweise spezielle Probeneingabemodule eingesetzt werden, welche besondere Vorrichtungen aufweisen (z.B. Anschlüsse für Kapillaren oder Spritzen), mittels deren die zu untersuchende Probenflüssigkeit in die Sensorkassette eingebracht werden kann. Weiterhin können auch spezielle Probeneingangs- und/oder Probenausgangsmodule eingesetzt werden, welche endständig in der Sensorkassette angeordnet sind und den Transport der Probenflüssigkeit oder anderer Funktionsfluide zu den innerhalb der Sensorkassette mittig angeordneten weiteren Modulen hin bzw. weg von diesen bewirken. Solche speziellen Probeneingangs- und/oder Probenausgangsmodule können somit als universell einsetzbare fluidische Kontaktstellen zum Analysator dienen, welche den Probenkanal der Sensorkassette mit einer entsprechenden Fluidik im Analysator verbinden, so dass alle weiteren (inneren) Module prinzipiell mit gleichen Abmessungen und Anschlüssen ausgestattet sein können, wodurch diese prinzipiell unbeschränkt kombiniert werden können.

## Patentansprüche

1. Sensorkassette (1) zum Einsetzen in einen Analysator, wobei die Sensorkassette (1) einen durchgehenden Messkanal (2) zur Aufnahme fluidischer Medien und sensorische Elemente (3, 4) zur Bestimmung von chemischen und/oder physikalischen Parametern der fluidischen Medien aufweist, **dadurch gekennzeichnet, dass** die Sensorkassette (1) zumindest zwei ohne Zerstörung untrennbar miteinander verbundene, jedoch separat hergestellte Sensormodule (5, 6, 6') aufweist, welche jeweils ein Gehäuse (7, 8) und einen Messkanalabschnitt (9, 10) aufweisen, der für den Durchfluss fluidischer Medien bestimmt ist, wobei die Messkanalabschnitte (9, 10) benachbarter Sensormodule (5, 6, 6') durch eine fluidische Kopplung (11) zum durchgehenden Messkanal (2) verbunden sind und wobei jedes Sensormodul ausgestattet ist, mit
• einem Trägerteil (16), auf dem ein Sensorarray mit zumindest zwei sensorischen Elementen (3, 4) aufgebracht ist,
• einem Abdeckteil, in dem der Messkanalabschnitt (9, 10) durchgängig ausgeformt ist,
• einem Dichtelement zur Abdichtung des Messkanals, wobei das Dichtelement zwischen dem Trägerteil (16) und dem Abdeckteil vorliegt oder am Abdeckteil angeformt ist,
• einer ersten Öffnung an einem Ende des Messkanalabschnitts (9, 10) zum Anschluss eines weiteren Moduls,
• einer zweiten Öffnung am anderen Ende des Messkanalabschnitts (9, 10) zum Anschluss an ein Analysegerät oder ein weiteres Modul,
wobei die Sensorkassette (1) ein Speicherelement (19) aufweist, auf welchem für die Sensorkassette (1) spezifische Informationen zu deren Aufbau aus den jeweiligen Sensormodulen (5, 6, 6'), gespeichert sind.

2. Sensorkassette (1) nach Anspruch 1, **dadurch gekennzeichnet, dass** die Sensorkassette (1) ein Dummymodul (5') aufweist, welches keine sensorischen Elemente, jedoch fluidische Anschlüsse an analogen Positionen wie das Sensormodul (5, 6, 6') aufweist.

3. Sensorkassette (1) nach Anspruch 1 oder 2, **dadurch gekennzeichnet, dass** die Sensorkassette (1) zumindest ein Sensormodul (6') aufweist, welches ein Sensorarray mit zumindest zwei sensorischen Elementen (3, 4) und einen freien Bereich (D) aufweist, der frei von sensorischen Elementen ist.

4. Sensorkassette (1) nach Anspruch 2 oder 3, **dadurch gekennzeichnet, dass** die Sensormodule (5, 6, 6') und die Dummymodule (5') einer Sensorkassette (1) gemeinsam die der Aufnahme des Analysators entsprechenden Abmessungen und Fluidanschlüsse (15) aufweisen.

5. Sensorkassette (1) nach einem der Ansprüche 1 bis 4, **dadurch gekennzeichnet, dass** die einzelnen Sensormodule (5, 6, 6') sowie ggf. Dummymodule (5') der Sensorkassette (1) durch eine feste mechanische Verschnappung, beispielsweise mittels Rastelementen (17), durch eine Verschweißung oder durch eine Verklebung miteinander verbunden sind.

6. Sensorkassette (1) nach einem der Ansprüche 1 bis 5, **dadurch gekennzeichnet, dass** die einzelnen Sensormodule (5, 6, 6') thermische Kontaktzonen aufweisen, über welche die Sensormodule (5, 6, 6') auf unterschiedliche Betriebstemperaturen thermostatisierbar sind.

7. Sensorkassette (1) nach Anspruch 6, **dadurch gekennzeichnet, dass** die thermischen Kontaktzonen benachbarter Sensormodule (5, 6, 6') der Sensorkassette (1) thermisch entkoppelt sind.

8. Sensorkassette (1) nach einem der Ansprüche 1 bis 7, **dadurch gekennzeichnet, dass** ein randseitiges Sensormodul (5, 6, 6') sowie ggf. ein randseitiges Dummymodul (5') der Sensorkassette (1) einen Griffteil (12) aufweist.

9. Sensorkassette (1) nach einem der Ansprüche 1 bis 8, **dadurch gekennzeichnet, dass** die auf dem Speicherelement (19) gespeicherten Informationen für die Sensorkassette (1) spezifische Informationen sind, welchen die Art der verwendeten Module (5, 5', 6, 6') und deren Anordnung bzw. Lage innerhalb der Sensorkassette (1) beschreiben.

10. Sensorkassette (1) nach einem der Ansprüche 1 bis 9, **dadurch gekennzeichnet, dass** auf dem Speicherelement (19) weiterhin Informationen gespeichert sind, welche die Art und Weise der Anordnung der einzelnen sensorischen Elemente (3, 4) bzw. freien Bereiche (D) innerhalb der jeweiligen Module (5, 6, 6') und/oder deren Nutzung beschreiben und insbesondere Informationen über Anordnung und/oder Nutzung elektrischer Kontaktstellen oder sonstige signalübertragender Bereiche, fluidischer Anschlüsse, thermischer Kontaktzonen und/oder fluidischer Steuerelemente innerhalb der einzelnen Module beinhalten.

11. Sensorkassette (1) nach einem der Ansprüche 1 bis 10, **dadurch gekennzeichnet, dass** auf dem Speicherelement (19) weiterhin Informationen gespeichert sind, welche die Art der einzelnen sensorischen Elemente (3, 4) und/oder deren Nutzung und Ansteuerung beschreiben und insbesondere Herstellinformationen, Kennliniendaten und/oder Kalibrationsinformationen oder Haltbarkeitsinformationen des jeweiligen sensorischen Elements (3, 4) beinhalten.

12. Sensorkassette (1) nach einem der Ansprüche 1 bis 11, **dadurch gekennzeichnet, dass** das Speicherelement (19) ein elektronisches Speicherelement, insbesondere ein Memory Chip oder eine Speicherkarte, ein RFID-Transponder, ein Magnetstreifen oder ein optischer Code, insbesondere ein ein- oder zweidimensionaler Barcode, ist.

13. Sensorkassette (1) nach einem der Ansprüche 1 bis 12, **dadurch gekennzeichnet, dass** das Speicherelement (19) fest mit der Sensorkassette (1) verbunden ist, insbesondere durch Aufbringen des Speicherelements (19) auf die Sensorkassette (1) oder Miteinbauen eines Speicherelements (19) in die Sensorkassette (1) bei Assemblieren dieser.

## Claims

1. A sensor cassette (1) for insertion into an analyzer, wherein the sensor cassette (1) has a continuous measuring channel (2) for receiving fluidic media and sensory elements (3, 4) for determining chemical and/or physical parameters of the fluidic media, **characterised in that** the sensor cassette (1) has at least two sensor modules (5, 6, 6') connected to one another, which are not separable from one another without destruction but are produced separately, which each have a housing (7, 8) and a measuring channel section (9, 10) which is determined for the through-flow of fluidic media, wherein the measuring channel sections (9, 10) of adjacent sensor modules (5, 6, 6') are connected to the continuous measuring channel by fluidic coupling (11) and wherein each sensor module is equipped with
• a carrier part (16) on which a sensor array with at least two sensory elements (3, 4) is attached,
• a cover part, in which the measuring channel section (9, 10) is continuously formed through,
• a sealing element for sealing the measuring channel, wherein the sealing element is provided between the carrier part (16) and the cover part or is integrally formed on the cover part,
• a first opening at one end of the measuring channel section (9, 10) for connecting a further module,
• a second opening at the other end of the measuring channel section (9, 10) for connection to an analysis device or a further module,
wherein the sensor cassette (1) comprises a storage element (19) on which specific information is stored for the sensor cassette (1) in respect of its composition from the respective sensor modules (5, 6, 6').

2. A sensor cassette (1) according to claim 1, **characterised in that** the sensor cassette (1) comprises a dummy module (5') which does not have any sensory elements, but fluidic connections at analogous positions such as the sensor module (5, 6, 6').

3. A sensor cassette (1) according to claim 1 or 2, **characterised in that** the sensor cassette (1) has at least one sensor module (6') which has a sensor array with at least two sensory elements (3, 4) and a free region (D) which is free of sensory elements.

4. A sensor cassette (1) according to claim 2 or 3, **characterised in that** the sensor modules (5, 6, 6') and the dummy modules (5') of a sensor cassette (1) jointly have the dimensions and fluid connections (15) corresponding to the receptacle of the analyzer.

5. A sensor cassette (1) according to one of claims 1 to 4, **characterised in that** the individual sensor modules (5, 6, 6') and optionally the dummy modules (5') of the sensor cassette (1) are connected to one another by a fixed mechanical snap-on connection, e.g. by means of detent elements (17), by welding or by gluing.

6. A sensor cassette (1) according to one of claims 1 to 5, **characterised in that** the individual sensor modules (5, 6, 6') have thermal contact zones, via which the sensor modules (5, 6, 6') can be thermostatted to different operating temperatures.

7. A sensor cassette (1) according to claim 6, **characterised in that** the thermal contact zones of adjacent sensor modules (5, 6, 6') of the sensor cassette (1) are thermally decoupled.

8. A sensor cassette (1) according to one of claims 1 to 7, **characterised in that** an edge-side sensor module (5, 6, 6') and optionally an edge-side dummy module (5') of the sensor cassette (1) have a handle part (12).

9. A sensor cassette (1) according to one of claims 1 to 8, **characterised in that** the information stored on the storage element (19) for the sensor cassette (1) is specific information which describes the type of the used modules (5, 5', 6, 6') and their arrangement or position within the sensor cassette (1).

10. A sensor cassette (1) according to one of claims 1 to 9, **characterised in that** information is further stored on the storage element (19) which describes the type and nature of the arrangement of the individual sensory elements (3, 4) or the free regions (D) within the respective modules (5, 6, 6') and/or their use, and in particular information about the arrangement and/or use of electrical contact points or other signal-transmitting regions, fluidic connections, thermal contact zones and/or fluidic control elements within the individual modules.

11. A sensor cassette (1) according to one of the claims 1 to 10, **characterised in that** information is further stored on the storage element (19) which describes the type of the individual sensory elements (3, 4) and/or their use and triggering, and in particular production information, characteristic data and/or calibration information or durability information of the respective sensory element (3, 4).

12. A sensor cassette (1) according to one of the claims 1 to 11, **characterised in that** the storage element (19) is an electronic storage element, in particular a memory chip or a memory card, an RFID transponder, a magnetic strip or an optical code, in particular a one- or two-dimensional barcode.

13. A sensor cassette (1) according to one of claims 1 to 12, **characterised in that** the storage element (19) is fixedly connected to the sensor cassette (1), in particular by attaching the storage element (19) to the sensor cassette (1) or by co-installing a storage element (19) in the sensor cassette (1) when assembling the latter.

## Revendications

1. Cassette de détection (1) destinée à être introduite dans un analyseur, la cassette de détection (1) présentant un canal de mesure continu (2), destiné à recevoir des milieux fluides, et des éléments capteurs (3, 4) pour déterminer des paramètres chimiques et/ou physiques des milieux fluides, **caractérisée en ce que** la cassette de détection (1) présente au moins deux modules de détection (5, 6, 6') reliés entre eux de manière indissociable sans destruction, mais réalisés séparément, lesquels présentent respectivement un boîtier (7, 8) et un tronçon de canal de mesure (9, 10) destiné au passage de milieux fluides, les tronçons de canal de mesure (9, 10) de modules de détection voisins (5, 6, 6') étant reliés au canal de mesure continu (2) par un couplage fluidique (11) et chaque module de détection étant équipé
• d'une partie de support (16) sur laquelle est montée une matrice de détection comportant au moins deux éléments capteurs (3, 4),
• d'une partie de recouvrement dans laquelle le tronçon de canal de mesure (9, 10) est réalisé en continu,
• d'un élément d'étanchéité pour rendre le canal de mesure étanche, l'élément d'étanchéité se trouvant entre la partie de support (16) et la partie de recouvrement ou étant formé sur la partie de recouvrement,
• d'une première ouverture à une extrémité du tronçon de canal de mesure (9, 10) pour raccorder un autre module,
• d'une deuxième ouverture à l'autre extrémité du tronçon de canal de mesure (9, 10) pour raccordement à un appareil d'analyse ou un autre module,
la cassette de détection (1) comportant un élément de mémoire (19) sur lequel sont stockées des informations spécifiques pour la cassette de détection (1) relatives à sa construction à partir des modules de détection respectifs (5, 6, 6').

2. Cassette de détection (1) selon la revendication 1, **caractérisée en ce que** la cassette de détection (1) comporte un module factice (5') qui comporte non pas des éléments capteurs, mais des raccords fluidiques à des positions analogiques telles que le module de détection (5, 6, 6').

3. Cassette de détection (1) selon la revendication 1 ou 2, **caractérisée en ce que** la cassette de détection (1) comporte au moins un module de détection (6') qui présente une matrice de détection avec au moins deux éléments capteurs (3, 4) et une zone libre (D) exempte d'éléments capteurs.

4. Cassette de détection (1) selon la revendication 2 ou 3, **caractérisée en ce que** les modules de détection (5, 6, 6') et les modules factices (5') d'une cassette de détection (1) présentent ensemble les dimensions et raccords pour fluides (15) appropriés pour loger l'analyseur.

5. Cassette de détection (1) selon l'une des revendications 1 à 4, **caractérisée en ce que** les différents modules de détection (5, 6, 6') et, le cas échéant, les modules factices (5') de la cassette de détection (1) sont reliés entre eux par un encliquetage mécanique fixe, par exemple, au moyen d'éléments d'encliquetage (17), par un soudage ou par un collage.

6. Cassette de détection (1) selon l'une des revendications 1 à 5, **caractérisée en ce que** les différents modules de détection (5, 6, 6') présentent des zones de contact thermiques via lesquelles les modules de détection (5, 6, 6') peuvent être thermostatés à différentes températures de service.

7. Cassette de détection (1) selon la revendication 6, **caractérisée en ce que** les zones de contact thermiques de modules de détection voisins (5, 6, 6') de la cassette de détection (1) sont découplées thermiquement.

8. Cassette de détection (1) selon l'une des revendications 1 à 7, **caractérisée en ce qu'**un module de détection de bord (5, 6, 6') ainsi que, le cas échéant, un module factice de bord (5') de la cassette de détection (1) présentent une partie poignée (12).

9. Cassette de détection (1) selon l'une des revendications 1 à 8, **caractérisée en ce que** les informations stockées sur l'élément de mémoire (19) sont des informations spécifiques pour la cassette de détection (1) qui décrivent le type des modules utilisés (5, 5', 6, 6') et leur agencement resp. position dans la cassette de détection (1).

10. Cassette de détection (1) selon l'une des revendications 1 à 9, **caractérisée en ce que** sont en outre stockées, sur l'élément de mémoire (19), des informations qui décrivent la manière dont les différents éléments capteurs (3, 4) resp. zones libres (D) sont agencés dans les modules respectifs (5, 6, 6') et/ou sont utilisés et qui contiennent plus particulièrement des informations sur l'agencement et/ou l'utilisation de points de contact électriques ou autres zones de transmission de signaux, de raccords fluidiques, de zones de contact thermiques et/ou d'éléments de commande fluidiques dans les différents modules.

11. Cassette de détection (1) selon l'une des revendications 1 à 10, **caractérisée en ce que** sont en outre stockées, sur l'élément de mémoire (19), des informations qui décrivent le type des différents éléments capteurs (3, 4) et/ou leur utilisation et commande et qui contiennent plus particulièrement des informations sur le fabricant, des données sur les courbes caractéristiques et/ou des informations sur l'étalonnage ou des informations sur la durée de vie de l'élément capteur respectif (3, 4).

12. Cassette de détection (1) selon l'une des revendications 1 à 11, **caractérisée en ce que** l'élément de mémoire (19) est un élément de mémoire électronique, en particulier une puce mémoire ou une carte mémoire, un transpondeur RFID, une bande magnétique ou un code optique, en particulier un code-barres unidimensionnel ou bidimensionnel.

13. Cassette de détection (1) selon l'une des revendications 1 à 12, **caractérisée en ce que** l'élément de mémoire (19) est relié à demeure à la cassette de détection (1), en particulier par montage de l'élément de mémoire (19) sur la cassette de détection (1) ou en introduisant également un élément de mémoire (19) dans la cassette de détection (1) lors de l'assemblage de celle-ci.
